# EUROPÄISCHE PATENTSCHRIFT

(11) **EP 1 035 918 B1**
(45) Veröffentlichungstag und Bekanntmachung des Hinweises auf die Patenterteilung: **12.02.2003**
(21) Anmeldenummer: 98958919.7
(22) Anmeldetag: 25.11.1998
(51) Int. Cl.: B01L 3/00, C12M 1/34

(54) **VORRICHTUNG ZUM MESSEN PHYSIOLOGISCHER PARAMETER**
DEVICE FOR MEASURING PHYSIOLOGICAL PARAMETERS
DISPOSITIF DE MESURE DE PARAMETRES PHYSIOLOGIQUES

(30) Priorität: 03.12.1997 DE 19753598
(43) Veröffentlichungstag der Anmeldung: 20.09.2000
(73) Patentinhaber: Micronas GmbH, 79108 Freiburg (DE)
(72) Erfinder: WOLF, Bernhard, D-79252 Stegen (DE); SIEBEN, Ulrich, D-79276 Reute (DE)
(74) Vertreter: Patentanwälte Westphal, Mussgnug & Partner
(86) Internationale Anmeldenummer: EP9807598
(87) Internationale Veröffentlichungsnummer: WO99028037

(56) Entgegenhaltungen:
- EP-A- 0 611 598
- WO-A-94/05414
- WO-A-97/44132
- WO-A-98/50154
- DE-A- 4 417 079
- US-A- 5 278 048

## Beschreibung

Die Erfindung betrifft eine Vorrichtung zum Messen physiologischer Parameter an in einer durch Wandungen begrenzten Prüfkammer in einem Nährmedium befindlichen biologischen Zellen.

Aus der DE-A-44 17 079 kennt man bereits eine Vorrichtung, die eine bis auf eine Einlaß- und eine Auslaßöffnung geschlossene Prüfkammer aufweist, in der tierische oder pflanzliche biologische Zellen in einem Nährmedium über einen längeren Zeitraum hinweg vital erhalten werden können. Dabei kann das Nährmedium durch die Ein- und AuslaßöffnungenbeispielsweisemittelseinesFluid-Handlingserneuert werden. Eine Wandung der Prüfkammer ist als Halbleiter-Chip ausgebildet, der eine der Prüfkammer zugewandte, Planarsensoren aufweisende aktive Seite hat, an der die in einem Nährmedium befindlichen biologischen Zellen adhärent angelagert sind. Der Halbleiter-Chip kann dazu beispielsweise an seiner aktiven Seite eine Oberflächenstrukturierung aufweisen, deren Rauhigkeit etwa der Rauhigkeit der Zellen entspricht, so daß diese den Halbleiter-Chip als Nachbarn akzeptieren und sich besser an ihm anlagern. Die Planarsensoren ermöglichen eine nahezu durchgehend ebene Chip-Oberfläche, so daß sich die Zellen unmittelbar an den Planarsensoren anlagern können. Dadurch ist es möglich, mit den Planarsensoren unmittelbar an den Zellen physikalische, elektrische oder elektrochemische Zellparameter, wie beispielsweise Ionenkonzentrationen, Gasgehalte, Zellpotentiale oder Zelltemperaturen zu messen. Somit kann die Reaktion der Zellen auf veränderte physikalische oder chemische Bedingungen, wie beispielsweise in dem Nährmedium enthaltene toxische Substanzen, Pharmaka, Umweltgifte und dergleichen auf einfache Weise getestet werden. Die Zellen wirken dabei als biologischer Vorverstärker, der die Meßempfindlichkeit der Planarsensoren erhöht. Mittels einer dem Halbleiter-Chip gegenüberliegenden Wandung zugeordneten Zusatz-Sensoren ist es außerdem möglich, globale physiologische Parameter in dem Nährmedium zu detektieren. Dadurch können zusätzliche Informationen über die Zellen gewonnen werden.

Obwohl sich die vorbekannte Vorrichtung in der Praxis bewährt hat, weist sie dennoch Nachteile auf. So ist sie noch vergleichsweise kompliziert aufgebaut. Die Vorrichtung ist deshalb relativ teuer, was deren Anwendungsmöglichkeiten einschränkt.

Aus US-A-52 78 048 ist ferner eine Vorrichtung der eingangs genannten Art bekannt, bei der eine Wandung der Prüfkammer durch einen Halbleiter-Chip aus Silizium gebildet ist, an dem biologische Zellen adhärent anlagert sind. Die dem Halbleiter-Chip gegenüberliegenden Wandung der Prüfkammer weist eine mit einer Indium-Zinnoxid-Schicht beschichtet Glasplatte auf, die in die Öffnung eines Gehäuseteils eingesetzt ist, das mittels eines ersten Dichtrings gegen die Glasplatte abgedichtet ist. Zwischen dem Gehäuseteil und dem Halbleiter-Chip ist ein zweiter Dichtring vorgesehen, der die Prüfkammer seitlich umgrenzt. Eine Halterung übergreift den Halbleiter-Chip und das Gehäuseteil außenseitig und hält diese Teile in Dichtlage. Die Halterung weist einen an dem Gehäuseteil angreifenden Messingflansch und eine damit über Haltestifte verbundene Messingplatte auf, auf welcher der Halbleiter-Chip aufliegt. Dabei dient die Messingplatte gleichzeitig als Anschlußkontakt für den Halbleiter-Chip, der in Kombination mit einer an der Einlaßöffnung vorgesehenen Referenzelektrode einen pH-Wert-Sensor bildet. Die Indium-Zinnoxid-Schicht der Glasplatte ist über einen Platin-Draht elektrisch kontaktiert und dient als Steuerelektrode.

Es besteht deshalb die Aufgabe, eine Vorrichtung der eingangs genannten Art zu schaffen, die einfach und kostengünstig aufgebaut ist und bei der die Abmessungen der Prüfkammer auf einfache Weise verändert werden können.

Diese Aufgabe wird mit den Merkmalen des Patentanspruchs 1 gelöst.

Dadurch ist es möglich, sämliche Sensoren der Vorrichtung in Halbleiter-Chips zu integrieren, wobei die Halbleiter-Chips gleichzeitig die Begrenzungswandungen der Prüfkammer bilden. In vorteilhafter Weise dient die zwischen den Halbleiter-Chips angeordnete Dichtung gleichzeitig auch als Abstandshalter, so daß zwischen den Halbleiter-Chips ein definiertes Kammervolumen gebildet ist, ohne daß in die Halbleiter-Chips Kavitäten aufweisen müssen. Die Halbleiter-Chips können somit als kostengünstige Planar-Chips ausgebildet sein. Vorteilhaft ist auch, daß der lichte Abstand zwischen den Halbleiter-Chips und somit die Höhe der Prüfkammer durch die Dicke der Dichtung definiert ist. Somit können Prüfkammern unterschiedlicher Höhe bzw. mit unterschiedlichen Abständen zwischen den Halbleiter-Chips mit den gleichen Halbleiter-Chips realisiert werden, wobei jeweils nur die Abmessungen der Dichtung und gegebenenfalls die der Halterung entsprechend angepaßt werden müssen. Somit können auf einfache Weise eine Vielzahl von Testkammern unterschiedlicher Abmessungen bereitgestellt werden. Gegebenfalls kann die Kammerhöhe der Vorrichtung auch nachträglich durch Austauschen der Dichtung gegen eine dickere oder dünnere Dichtung verändert werden. So erfordern beispielsweise bestimmte experimentelle Techniken, wie die Bestimmung der metabolischen Aktivität von Zellen durch Messung des ph-Werts und der Sauerstoff-Konzentration, ein hohes Zellenzahl- zu Kammervolumen-Verhältnis beziehungsweise eine extrem geringe Kammerhöhe. Bei anderen Anwendungen kann dagegen eine größere Kammerhöhe vorteilhaft sein, beispielsweise um die durch eine Nährflüssigkeitsströmung auf die Zellen ausgeübten Strömungs-Scherkräfte zu reduzieren. Die an der Halterung vorgesehenen, mit den Anschlußkontakten der Halbleiter-Chips zusammenwirkenden Gegenkontakte ermöglichen auf einfache Weise eine elektrische Verbindung zwischen den die Prüfkammer begrenzenden Halbleiter-Chips und/oder den Halbleiter-Chips und einer externen Auswerteeinheit oder einer Stromversorgungseinheit. Insgesamt ergibt sich somit ein einfach aufgebauter, kostengünstig herstellbarer und leicht handhabbarer Biomonitor.

Vorteilhaft ist, wenn wenigstens ein die Einlaßöffnung aufweisender Einlaßkanal und/oder wenigstens ein die Auslaßöffnung aufweisender Auslaßkanal die Dichtung durchsetzt. Die Ein- und Auslaßkanäle der Prüfkammer sind also in der Dichtung angeordnet, so daß Wandungsdurchbrüche in den Halbleiter-Chips entfallen können.

Zweckmäßigerweise besteht die Dichtung aus einem elastischen Material und ist vorzugsweise als O-Ring ausgebildet. Als Ein- oder Auslaßkanal kann dann beispielsweise eine Hohlnadel vorgesehen sein, welche die Dichtung durchsetzt. Die Vorrichtung ist dann besonders flexibel handhabbar.

Zweckmäßig ist, wenn die Klemmteile als Klemmschenkel ausgebildet sind, die gegen die Rückstellkraft ihres Werkstoffs federnd elastisch voneinander wegverformbar sind. Die Halteklemme oder die Halteklammer ist dann besonders einfach aufgebaut und kann beispielsweise als im Querschnitt U-förmige Klemme und/oder als Klemmleiste ausgebildet sein. Die Klemmschenkel können einstückig miteinander verbunden sein.

Besonders vorteilhaft ist, wenn die Gegenkontakte an dem Klemmteil befestigt sind. Die Rückstellkraft der Halteklemme oder Halteklammer kann dann sowohl zum Andrücken der Gegenkontakte an die Anschlußkontakte der Halbleiter-Chips, als auch zum Andrücken der Halbleiter-Chips an die dazwischen befindliche Dichtung genutzt werden. Gegebenenfalls kann an einem Klemmteil der Halterung ein Leitgummi vorgesehen sein, der den elektrischen Kontakt zu den an den Halbleiter-Chips befindlichen Anschlußkontakten herstellt.

Eine bevorzugte, besonders vorteilhafte Ausführungsform der Erfindung sieht vor, daß der zum adhärenten Anlagern der biologischen Zellen vorgesehene erste Halbleiter-Chip wenigstens ein Array mit mehreren, voneinander beabstandeten Planarsensoren aufweist. Die Vorrichtung ermöglicht dann eine ortsaufgelöste Messung an einzelnen Zellen einer an der aktiven Seite des Halbleiter-Chips adhärent angelagerten Zellkultur.

Vorteilhaft ist, wenn wenigstens ein Halbleiter-Chip zumindest eine Mikro-Pumpe aufweist, die wenigstens einen, mit einem Einlaßkanal oder einem Auslaßkanal der Prüfkammer verbundenen Pumpenanschluß hat. Mit der Mikropumpe kann dann beispielsweise das Nährmedium in der Prüfkammer umgefördert werden.

Zweckmäßigerweise weist wenigstens ein Halbleiter-Chip zum Temperieren der Prüfkammer an seiner der Prüfkammer abgewandten Außenseite eine in den Halbleiter-Chip integrierte oder an diesem befestigte Elektroheizung auf. Dieser kann beispielsweise an der aktiven Seite des Halbleiter-Chips ein Temperaturfühler sowie eine gegebenenfalls in den Halbleiter-Chip integrierte Regeleinrichtung zugeordnet sein, welche die Temperatur in der Prüfkammer auf einen vorgegebenen Wert, beispielsweise 37°C regelt. Die Elektroheizung kann beispielsweise ein auf die Rückseite des Halbleiter-Chips aufgedampfter Platin-Mänder sein.

Eine Ausführungsform der Erfindung sieht vor, daß die Vorrichtung zur Übertragung von Meßdaten an eine externe Auswerteeinrichtung einen Transponder aufweist. Die Meßdaten können dann drahtlos an die Auswerteeinrichtung übertragen werden, wodurch die Vorrichtung insbesondere an schwer zugänglichen oder hermetisch abgeschirmten Stellen besser verwendbar ist. Auch ist die Handhabung der Vorrichtung erleichtert.

Besonders vorteilhaft ist, wenn die beiden einander gegenüberliegend angeordneten Halbleiter-Chips identisch aufgebaut sind. Die Vorrichtung ist dann noch kostengünstiger herstellbar.

Eine bevorzugte Ausführungsform der Erfindung sieht vor, daß der erste Halbleiter-Chip ein optisches Fenster aufweist, an dessen der Prüfkammer zugewandter Innenseite biologische Zellen adhärent anlagerbar sind. Das Fenster schließt dann mit seiner der Prüfkammer zugewandten Innenseite vorzugsweise bündig an den die Planarsensoren aufweisenden aktiven Bereich des Halbleiter-Chips an, so daß sich eine an der aktiven Seite des Halbleiter-Chips angelagerte Zellkultur bis über das optische Fenster erstrecken kann. Dadurch ist es möglich, während der mittels der Sensoren durchgeführten Messungen die biologischen Zellen beispielsweise mit einem Mikroskop oder einer Kamera zu beobachten. Dadurch können zusätzliche Informationen über die Zellen gewonnen werden, die gegebenenfalls mit von den Sensoren gelieferten Meßwerten korreliert werden können. Durch das Fenster können die Zellen aber auch mit Licht, beispielsweise mit Laserlicht bestrahlt werden, um diese zu stimulieren. Die Reaktion der Zellen kann dann gegebenfalls mittels der Sensoren getestet werden. Selbstverständlich kann das Fenster aber auch für andere optische Untersuchungen, wie beispielsweise Spektralmessungen, verwendet werden. Wenn auch der zweite Halbleiter-Chip wenigstens ein optisches Fenster aufweist kann an das Fenster des einen Halbleiter-Chips eine Kondensorlinse und an das Fenster des anderen Halbleiter-Chips ein Objektiv herangeführt werden. Die Prüfkammer kann dann noch besser mikroskopiert werden.

Eine vorteilhafte Ausführungsform der Erfindung sieht vor, daß an der aktiven Seite des ersten Halbleiter-Chips einen Wirkstoff produzierende Zellen adhärent anlagerbar oder angelagert sind. So können beispielsweise Insulin produzierende Zellen an dem ersten Halbleiter-Chip angelagert sein. Die Vorrichtung kann dann beispielsweise in einem menschlichen oder tierischen Körper implantiert werden, um dort über einen längeren Zeitraum hinweg, Insulin zu produzieren. Dabei kann das Insulin gegebenenfalls mittels einer in die Vorrichtung integrierten Mikropumpe kontrolliert an den Körper abgegeben werden. In vorteilhafter Weise kann die Vitalität der Zellen und somit deren Fähigkeit Insulin zu produzieren mittels der Sensoren überwacht werden.

Nachfolgend sind Ausführungsbeispiele der Erfindung anhand der Zeichnungen näher erläutert: Es zeigen zum Teil stärker schematisiert:
- Fig.1: eine Aufsicht auf die in Fig.2 mit I markierte Längsmittelebene, welche den die Planarsensoren aufweisenden ersten Halbleiter-Chip sowie das in diesen eingesetzte optische Fenster besonders gut erkennen läßt,
- Fig.2: einen Querschnitt durch die in Fig.1 mit II bezeichnete Ebene, welche die seitlich durch den Dichtring sowie ober- und unterseitig jeweils durch Halblexter-Chips begrenzte Meßkammer mit den darin befindlichen Zellen erkennen läßt,
- Fig.3: eine Darstellung ähnlich Fig.1, wobei die Dichtung ein elastischer O-Ring ist, durch den Ein- und Auslaßkanäle bildende Hohlnadeln hindurchgesteckt sind,
- Fig.4: eine Ansicht auf die in Fig.3 mit IV bezeichnete Querschnittsebene und
- Fig.5: einen Teilquerschnitt durch einen Halbleiter-Chip, an dessen aktiver Flachseite biologische Zellen adhärent angelagert sind.

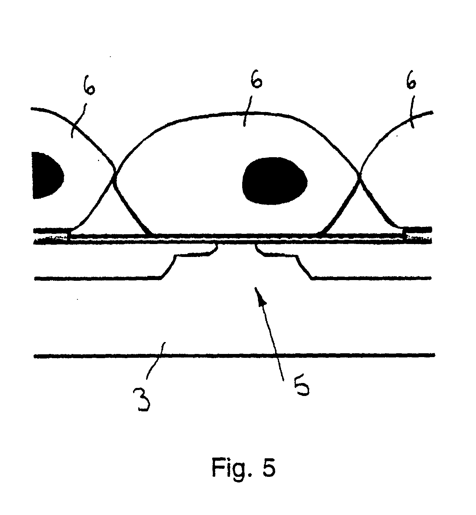

Eine im ganzen mit 1 bezeichnete Vorrichtung zum Messen physiologischer Parameter weist eine bis auf eine Einlaßöffnung und eine Auslaßöffnung für Nährmedium geschlossene Prüfkammer auf, die unterseitig durch einen ersten Halbleiter-Chip 3 und oberseitig durch einen zweiten Halbleiter-Chip 4 begrenzt ist. Der unterseitig angeordnete Halbleiter-Chip 3 weist an seiner der Prüfkammer 2 zugewandten aktiven Seite eine Vielzahl von Planarsensoren 5 auf, die in das Substrat des Halbleiter-Chips 3 integriert sind (Fig.5). Der Halbleiter-Chip 3 weist an seiner aktiven Seite eine ebene Oberfläche auf.

Wie aus Fig.2 und 4 besonders gut erkennbar ist, sind an der aktiven Seite des Halbleiter-Chips 3 in einem Nährmedium biologische Zellen 6 anlagerbar, wie beispielsweise Meeresalgen oder Fischkiemen. Der Halbleiter-Chip 3 weist dazu an seiner der Prüfkammer 2 zugewandten Flachseiteeine Oberflächenstrukturierung auf, deren Rauhigkeit etwa der Rauhigkeit der anzulagernden Zellen entspricht, so daß diese den Halbleiter-Chip als Nachbarn akzeptieren. In Fig. 5 ist am Beispiel eines als Feldeffekttransitor ausgebildeten, in das Substrat des Halbleiter-Chips 3 integrierten Planarsensors 5 erkennbar, daß die Zellen 6 unmittelbar benachbart zu den Planarsensoren 5 angeordnet sind. Dabei beträgt der Abstand zwischen den Zellen und den aktiven Bereichen der Planarsensoren typischerweise etwa 20 Nanometer. Dadurch ist es möglich, mittels der Planarsensoren 5 direkt an den einzelnen Zellen physiologische Parameter, wie beispielsweise Ionenkonzentrationen zu messen.

Auch der zweite Halbleiter-Chip 4 weist eine der Prüfkammer 2 zugewandte aktive Seite auf. Diese hat mehrere Zusatz-Sensoren, die ebenfalls als Planarsensoren ausgebildet sind. Mit den Zusatz-Sensoren können globale physiologische Parameter in einem in der Prüfkammer 2 enthaltenen Nährmedium gemessen werden.

Zwischen den Halbleiter-Chips 3, 4 ist als Abstandshalter eine Dichtung 7 angeordnet, die an beiden Halbleiter-Chips 3, 4 anliegt und die Prüfkammer 2 umgrenzt. Die Halbleiter-Chips 3, 4 und die dazwischen befindliche Dichtung 7 sind mittels einer die Halbleiter-Chips 3, 4 außenseitig übergreifenden Halterung 8 in Dichtlage gehalten.

Die Halbleiter-Chips 3,4 weisen jeweils an ihrer der Prüfkammer 2 abgewandten Außenseite elektrische Anschlußkontakte auf, die mit Gegenkontakten der Halterung 9 kontaktieren. Zur elektrischen Verbindung der beiden Halbleiter-Chips 3, 4 weist die Halterung 8 mit Gegenkontakten verbundene Verbindungsleitungen auf, die in die Halterung integriert sind. Zur Stromversorgnung der Halbleiter-Chips 3, 4 und zur Übertragung von Meß- und Steuerdaten hat die Halterung 8 einen Anschluß für ein externes mit Gegenkontakten der Halterung 8 verbundenes Anschlußkabel 9.

Insgesamt ergibt sich somit eine einfach aufgebaute und kostengünstig herstellbare Vorrichtung 1, mit der sowohl unmittelbar an den Zellen physiologische Parameter, als auch globale physiologische Parameter des Nährmediums registriert werden können.

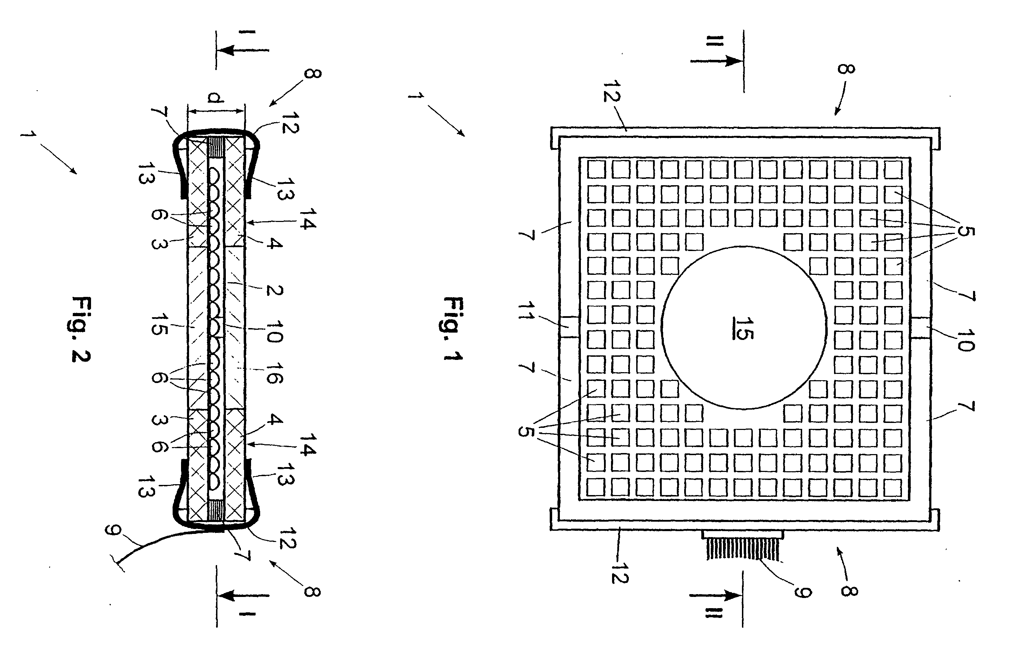

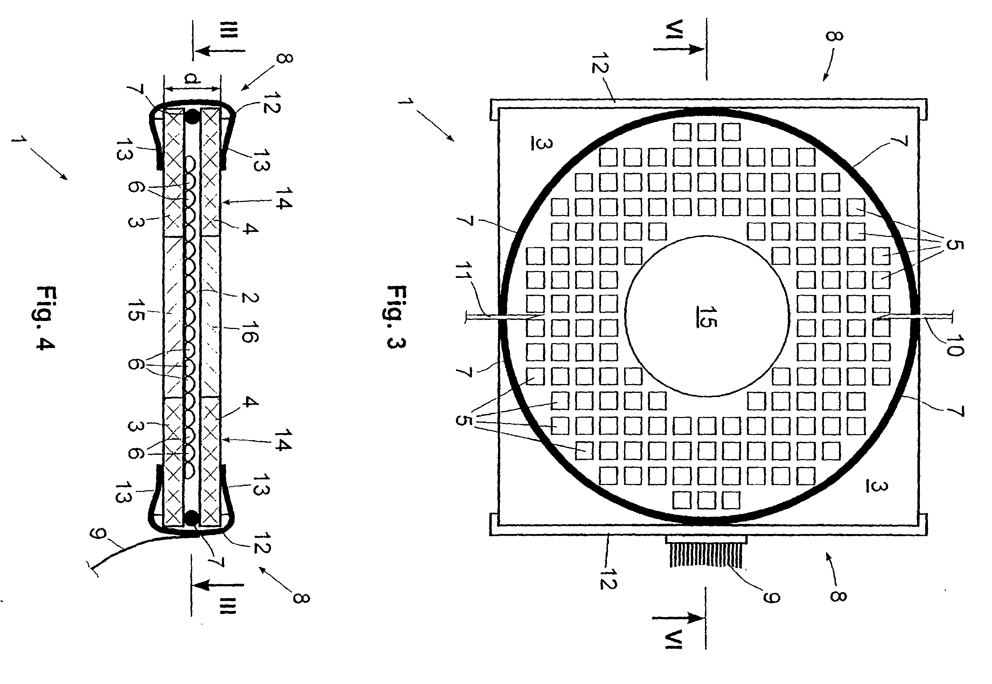

Bei den in Fig.1 und 2 gezeigten Ausführungsbeispielen ist die Dichtung 7 als Silikondichtung ausgebildet, die von einem Einlaßkanal 10 und einem Auslaßkanal 11 durchsetzt ist. An diesen kann beispielsweise ein Fluid-Handling zum Erneuern eines in der Prüfkammer 2 enthaltenen Nährmediums angeschlossen werden. Bei dem Ausführungsbeispiel nach Fig. 3 und 4 ist die Dichtung als elastischer O-Ring ausgebildet, der von zwei Hohlnadeln durchsetzt ist, welche die Ein- und Auslaßkanäle 10, 11 bilden. Die Vorrichtung 1 ist dann noch besser handhabbar, wobei die Hohlnadeln je nach Applikation unterschiedlich angeordnet sein können und/oder unterschiedliche Durchmesser aufweisen können.

Die Halterung 8 hat zwei im Querschnitt etwa U-förmige Halteklemmen 12, die jeweils zwei Klemmschenkel 13 aufweisen, die in Ausgangslage einen geringeren lichten Abstand haben, als die Gesamtdicke d der zwischen den Klemmschenkel 13 einzuspannenden, durch die Dichtung 7 voneinander beabstandeten Halbleiter-Chips 3, 4. Aus der Ausgangslage sind die Klemmschenkel 13 gegen die Rückstellkraft ihres Werkstoffs voneinander wegbewegbar. Dadurch können die Halterung 8 und die Halbleiter-Chips 3, 4 mit Dichtungen 7 unterschiedlicher Dicke kombiniert werden. Somit können je nach Meßaufgabe auf einfache Weise unterschiedliche lichte Abstände zwischen den Halbleiter-Chips 3, 4 eingestellt werden, ohne daß dazu Modifikationen an den Halbleiter-Chips 3, 4 oder der Halterung 8 erforderlich sind. Als Dichtungen können beispielsweise O-Ringe vorgesehen sein, die in unterschiedlichsten Abmessungen im Handel verfügbar sind.

Die Gegenkontakte sind innenseitig an den Klemmschenkeln 13 befestigt und liegen in Klemmstellung der Klemmschenkel 13 an den Anschlußkontakten an, die an den einander abgewandten äußeren Flachseiten der Halbleiter-Chips 3, 4 angeordnet und mit Durchkontaktierungen mit den Planarsensoren 5 beziehungsweise den Zusatz-Sensoren verbunden sind. Die Klemmkraft der Halterung 8 drückt somit sowohl die Gegenkontakte an die Anschlußkontakte, als auch die Halbleiter-Chips 3, 4 an die Dichtung 7 an.

Wie aus Figur 1 und 3 erkennbar ist, weist der zum adhärenten Anlagern der biologischen Zellen 6 vorgesehene erste Halbleiter-Chip 3 ein Array mit einer Vielzahl voneinander beabstandeter Planarsensoren 5 auf. Zum Messen unterschiedlicher physiologischer Parameter weist das Array jeweils mehrere unterschiedliche Planarsensoren 5 auf. Die Vorrichtung 1 ermöglicht dadurch eine ortsaufgelöste Messung an den biologischen Zellen 6, wobei mehrere unterschiedliche Parameter gleichzeitig registriert werden können.

Wie aus Figur 1 erkennbar ist, hat der erste Halbleiter-Chip 3, benachbart zu seinem die Planarsensoren 5 aufweisenden aktiven Bereich ein optisches Fenster 15, das in eine Lochung in dem Substrat des Halbleiter-Chips 3 eingesetzt ist. Die der Prüfkammer 2 zugewandte Innenseite des Fensters 15 bildet mit der aktiven Innenseite des Halbleiter-Chips 3 eine Ebene, an der biologische Zellen 6 adhärent anlagerbar sind. In Figur 2 und 4 ist deutlich zu erkennen, daß eine an dem Halbleiter-Chip 3 angelagerte Zellkultur sowohl an dem aktiven Bereich des Halbleiter-Chips 3, als auch an dem Fenster 15 angelagert ist. Auch der zweite Halbleiter-Chip 4 weist ein optisches Fenster 16 auf. Mittels der Fenster 15, 16 können in der Prüfkammer 2 befindliche Zellen mikroskopiert werden, indem beispielsweise an eines der Fenster 15, 16 ein Objektiv und an das andere Fenster 16, 15 eine Kondensorlinse herangeführt wird.

Insgesamt erigibt sich somit eine Vorrichtung 1 zum Messen physiologischer Parameter, die eine Prüfkammer 2 hat, die durch einen ersten Halbleiter-Chip 3 und einen zweiten Halbleiter-Chip 4 begrenzt ist, zwischen denen als Abstandshalter eine die Prüfkammer 2 umgrenzende Dichtung 7 angeordnet ist. Der erste Halbleiter-Chip 3 hat eine der Prüfkammer 2 zugewandte, Planarsensoren aufweisende aktive Seite, an der in einem Nährmedium befindliche biologische Zellen 6 adhärent anlagerbar sind, um physiologische Parameter unmittelbar an den Zellen 6 zu messen. Der zweite Halbleiter-Chip 4 weist an einer aktiven, der Prüfkammer 2 zugewandten Seite wenigstens einen Zusatz-Sensor zum messen globaler physiologischer Parameter auf. Die Halbleiter-Chips 3, 4 sind mittels einer diese außenseitig übergreifenden Halterung 8 in Dichtlage gehalten. Außerhalb der Prüfkammer 2 weisen die Halbleiter-Chips 3, 4 jeweils elektrische Anschlußkontakte auf, die mit Gegenkontakten der Halterung kontaktieren. Die Vorrichtung ist einfach und kostengünstig aufgebaut.

## Patentansprüche

1. Vorrichtung zum Messen physiologischer Parameter, mit einer bis auf wenigstens eine Einlaßöffnung und zumindest eine Auslaßöffnung geschlossenen Prüfkammer (2), wobei zwei einander gegenüberliegende Wandungen der Prüfkammer als Halbleiter-Chips (3, 4) ausgebildet sind, wobei zumindest ein erster dieser Halbleiter-Chips (3, 4) eine der Prüfkammer (2) zugewandte, Planarsensoren (5) aufweisende aktive Seite hat, an der in einem Nährmedium befindliche biologische Zellen (6) adhärent anlagerbar sind, zur Messung physiologischer Parameter unmittelbar an den Zellen (6), wobei der diesem Halbleiter-Chip (3) gegenüberliegende zweite Halbleiter-Chip (4) an seiner aktiven, der Prüfkammer (2) zugewandten Seite wenigstens einen Zusatz-Sensor zur Messung globaler physiologischer Parameter aufweist, wobei als Abstandshalter zwischen den Halbleiter-Chips (3, 4) eine die Prüfkammer (2) umgrenzende Dichtung (7) angeordnet ist, wobei die Halbleiter-Chips (3, 4) mittels einer diese außenseitig übergreifenden Halterung (8) in Dichtlage gehalten sind, wobei die Halbleiter-Chips (3, 4) außerhalb der Prüfkammer (2) jeweils elektrische Anschlußkontakte aufweisen, die mit Gegenkontakten der Halterung (8) kontaktieren, wobei die Halterung (8) zumindest eine Halteklemme (12) oder eine Halteklammer mit wenigstens zwei Klemmteilen aufweist, die in Ausgangslage einen geringeren lichten Abstand haben, als die Gesamtdicke (d) der zwischen den Klemmteilen einzuspannenden, durch die Dichtung (7) voneinander beabstandeten Halbleiter-Chips (3, 4), und wobei die Klemmteile gegen eine Rückstellkraft voneinander wegbewegbar sind.

2. Vorrichtung nach Anspruch 1, wobei wenigstens ein die Einlaßöffnung aufweisender Einlaßkanal (10) und/oder wenigstens ein die Auslaßöffnung aufweisender Auslaßkanal (11) die Dichtung (7) durchsetzt.

3. Vorrichtung nach Anspruch 1 oder 2, wobei die Dichtung (7) aus elastischem Material besteht und vorzugsweise ein O-Ring ist.

4. Vorrichtung nach einem der Ansprüche 1 bis 3, wobei die Klemmteile als Klemmschenkel (13) ausgebildet sind, die gegen die Rückstellkraft ihres Werkstoffs federnd elastisch voneinander wegverformbar sind.

5. Vorrichtung nach einem der Ansprüche 1 bis 4, wobei die Gegenkontakte an dem Klemmteil befestigt sind.

6. Vorrichtung nach einem der Ansprüche 1 bis 5, wobei der zum adhärenten Anlagern der biologischen Zellen vorgesehene erste Halbleiter-Chip (3) wenigstens ein Array mit mehreren, voneinander beabstandeten Planarsensoren (5) aufweist.

7. Vorrichtung nach einem der Ansprüche 1 bis 6, wobei wenigstens ein Halbleiter-Chip (3, 4) zumindest eine Mikropumpe aufweist, die wenigstens einen, mit einem Einlaßkanal (10) oder einem Auslaßkanal (11) der Prüfkammer (2) verbundenen Pumpenanschluß hat.

8. Vorrichtung nach einem der Ansprüche 1 bis 7, wobei wenigstens ein Halbleiter-Chip (3, 4) zum Temperieren der Prüfkammer (2) an seiner der Prüfkammer (2) abgewandten Außenseite eine in den Halbleiter-Chip (3, 4) integrierte oder an diesem befestigte Elektroheizung aufweist.

9. Vorrichtung nach einem der Ansprüche 1 bis 8, wobei zur Übertragung von Meßdaten an eine externe Auswerteeinrichtung ein Transponder vorgesehen ist.

10. Vorrichtung nach einem der Ansprüche 1 bis 9, wobei die beiden einander gegenüberliegend angeordneten Halbleiter-Chips (3, 4) identisch aufgebaut sind.

11. Vorrichtung nach einem der Ansprüche 1 bis 10, wobei der erste Halbleiter-Chip (3) ein optisches Fenster (15) aufweist, an dessen der Prüfkammer (2) zugewandter Innenseite biologische Zellen (6) adhärent anlagerbar sind.

12. Vorrichtung nach einem der Ansprüche 1 bis 11, wobei der zweite Halbleiter-Chip (4) wenigstens ein optisches Fenster (16) aufweist.

13. Vorrichtung nach einem der Ansprüche 1 bis 12, wobei als Stromversorgung ein elektrischer Energiespeicher und/oder eine Solarzelle und/oder außenseitig angeordnete Elektroden zur galvanischen Stromerzeugung vorgesehen ist.

14. Vorrichtung nach einem der Ansprüche 1 bis 13, wobei an der aktiven Seite des ersten Halbleiter-Chips (3) einen Wirkstoff produzierende biologische Zellen (6) adhärent anlagerbar oder angelagert sind.

## Revendications

1. Dispositif pour mesurer des paramètres physiologiques comprenant une chambre d'essai (2) fermée à l'exception d'au moins un orifice d'entrée et d'au moins un orifice de sortie,
deux parois opposées de la chambre d'essai étant constituées par des puces semi-conductrices (3, 4), dont au moins une première des puces semi-conductrices (3, 4) comporte un côté actif muni de capteurs planaires (5), tourné vers la chambre d'essai (2), et sur lequel pouvant s'accumuler par adhérence des cellules biologiques (6) dans un milieu nutritif, pour mesurer les paramètres physiologiques directement sur les cellules (6), tandis que la seconde puce semi-conductrice (4) en regard de cette puce semi-conductrice (3), présente sur son côté actif tourné vers la chambre d'essai (2), au moins un capteur complémentaire pour mesurer les paramètres physiologiques globaux, dispositif dans lequel
un joint (7) délimitant la chambre d'essai (2) est prévu comme organe d'écartement entre les puces semi-conductrices (3, 4),
les puces semi-conductrices (3, 4) sont maintenues en position d'étanchéité à l'aide d'un support (8) qui les entoure du côté extérieur,
les puces semi-conductrices (3, 4) ayant à l'extérieur de la chambre d'essai (2), chaque fois des contacts de branchement électriques mis en contact avec des contacts complémentaires du support (8), et
le support (8) au moins une pince de fixation (12) ou une pince à au moins deux parties de pince qui, en position de repos, ont un intervalle libre plus faible que l'épaisseur totale (d) des puces semi-conductrices (3, 4) écartées l'une de l'autre par le joint (7) et qui sont à serrer entre les parties de pince, et
les parties de pince peuvent s'écarter contre une force de rappel.

2. Dispositif selon la revendication 1,
dans lequel le joint (7) est traversé par au moins un canal d'entrée (10) comportant un orifice d'entrée et/ou par au moins un canal de sortie (11) comportant un orifice de sortie.

3. Dispositif selon la revendication 1 ou 2,
dans lequel le joint (7) est en matière élastique et est constitué de préférence par un joint torique.

4. Dispositif selon l'une quelconque des revendications 1 à 3,
dans lequel les parties de pince sont réalisées sous la forme de branches de pince (13) qui peuvent être déformées élastiquement comme des ressorts les unes des autres contre la force de rappel de leur matière.

5. Dispositif selon l'une quelconque des revendications 1 à 4,
dans lequel les contre-contacts sont fixés sur la partie de pince.

6. Dispositif selon l'une quelconque des revendications 1 à 5,
dans lequel la première puce semi-conductrice (3) prévue pour l'accumulation par adhérence de cellules biologiques, comporte au moins un réseau de plusieurs capteurs planaires (5) éloignés les uns des autres.

7. Dispositif selon l'une quelconque des revendications 1 à 6,
dans lequel au moins une puce semi-conductrice (3, 4) comporte au moins une micro-pompe qui possède au moins un branchement de pompe relié à un canal d'entrée (10) ou à un canal de sortie (11) de la chambre d'essai (2).

8. Dispositif selon l'une quelconque des revendications 1 à 7,
dans lequel au moins une puce semi-conductrice (3, 4) comporte sur son côté extérieur à l'opposé de la chambre d'essai (2), un chauffage électrique intégré à la puce semi-conductrice (3, 4) ou fixé à celle-ci pour mettre en température la chambre d'essai (2).

9. Dispositif selon l'une quelconque des revendications 1 à 8,
dans lequel, il est prévu un transpondeur pour transmettre les données de mesure vers une installation d'exploitation externe.

10. Dispositif selon l'une quelconque des revendications 1 à 9, dans lequel les deux puces semi-conductrices (3, 4), opposées l'une à l'autre sont de construction identique.

11. Dispositif selon l'une quelconque des revendications 1 à 10,
dans lequel la première puce semi-conductrice (3) comporte une fenêtre optique (15) dont le côté intérieur tourné vers la chambre d'essai (2) permet l'accumulation par adhérence de cellules biologiques (6).

12. Dispositif selon l'une quelconque des revendications 1 à 11,
dans lequel la seconde puce semi-conductrice (4) comporte au moins une fenêtre optique (16).

13. Dispositif selon l'une quelconque des revendications 1 à 12,
dans lequel comme alimentation électrique, il est prévu un accumulateur d'énergie électrique et/ou une cellule solaire et/ou des électrodes prévues sur le côté extérieur pour générer du courant par voie galvanique.

14. Dispositif selon l'une quelconque des revendications 1 à 13,
dans lequel le côté actif de la première puce semi-conductrice (3) reçoit par accumulation par adhérence ou dépôt des cellules biologiques (6) produisant une matière active.

## Claims

1. A device for measuring physiological parameters, having a test chamber (2) sealed except for at least one inlet opening and at least one outlet opening, wherein two mutually opposing walls of the test chamber take the form of semiconductor chips (3, 4), wherein at least a first one of these semiconductor chips (3, 4) has an active side facing the test chamber (2) and comprising planar sensors (5), to which side biological cells (6) located in a nutrient medium may be adhesively attached for measuring physiological parameters directly at the cells (6), wherein the second semiconductor chip (4) opposite this semiconductor chip (3) comprises at least one additional sensor on its active side facing the test chamber (2) for measuring overall physiological parameters, wherein a seal (7) surrounding the test chamber (2) is arranged as a spacer between the semiconductor chips (3, 4), wherein the semiconductor chips (3, 4) are held in a sealed position by means of a holding means (8) clasping them on the outside, wherein the semiconductor chips (3, 4) each comprise electrical terminal contacts outside the test chamber (2), which terminal contacts make contact with counter-contacts on the holding means (8), wherein the holding means (8) comprises at least one holding clamp (12) or holding clip with at least two clamping members which have a smaller space between them in the starting position than the total thickness (d) of the semiconductor chips (3, 4) to be clamped between the clamping members and spaced from one another by the seal (7), and wherein the clamping members may be moved apart against a restoring force.

2. A device according to claim 1, wherein at least one inlet channel (10) comprising the inlet opening and/or at least one outlet channel (11) comprising the outlet opening passes through the seal (7).

3. A device according to claim 1 or claim 2, wherein the seal (7) consists of resilient material and preferably is an O-ring.

4. A device according to one of claims 1 to 3, wherein the clamping members take the form of clamping legs (13), which may be deformed resiliently and in spring-loaded manner away from one another against the restoring force of their material.

5. A device according to one of claims 1 to 4, wherein the counter-contacts are attached to the clamping member.

6. A device according to one of claims 1 to 5, wherein the first semiconductor chip (3) provided for adhesive attachment of the biological cells comprises at least one array having a plurality of mutually spaced planar sensors (5).

7. A device according to one of claims 1 to 6, wherein at least one semiconductor chip (3, 4) comprises at least one micropump, which has at least one pump connection connected to an inlet channel (10) or an outlet channel (11) of the test chamber (2).

8. A device according to one of claims 1 to 7, wherein at least one semiconductor chip (3, 4) comprises an electric heating means on its outer side remote from the test chamber (2) for stabilising the temperature of the test chamber (2), which electric heating means is incorporated into the semiconductor chip (3, 4) or attached thereto.

9. A device according to one of claims 1 to 8, wherein a transponder is provided for transmission of measured data to an external evaluation means.

10. A device according to one of claims 1 to 9, wherein the two mutually opposing semiconductor chips (3, 4) are of identical construction.

11. A device according to one of claims 1 to 10, wherein the first semiconductor chip (3) comprises an optical window (15), to the inside of which facing the test chamber (2) biological cells (6) may be adhesively attached.

12. A device according to one of claims 1 to 11, wherein the second semiconductor chip (4) comprises at least one optical window (16).

13. A device according to one of claims 1 to 12, wherein an electrical energy store and/or a solar cell and/or externally arranged electrodes for electrochemical power generation is/are provided as power supply.

14. A device according to one of claims 1 to 13, wherein biological cells (6) producing an active substance may be attached or are attached adhesively to the active side of the first semiconductor chip (3).
